# EUROPÄISCHE PATENTSCHRIFT

(11) **EP 2 737 852 B1**
(45) Veröffentlichungstag und Bekanntmachung des Hinweises auf die Patenterteilung: **19.08.2015**
(21) Anmeldenummer: 12195054.7
(22) Anmeldetag: 30.11.2012
(51) Int. Cl.: A61B 6/00, G01N 23/04, A61B 6/03, G01T 7/00

(54) **Verfahren zum Erfassen geometrischer Abbildungseigenschaften eines Flachbilddetektors, entsprechend eingerichtete Röntgenprüfanlage und Kalibrierkörper**
Method for detecting the geometric imaging properties of a flat screen detector, accordingly designed x-ray test system and calibration body
Procédé de détection des propriétés d'imagerie géométriques d'un détecteur à panneau plat, systèm de test adapté suivant et corps de calibrage

(43) Veröffentlichungstag der Anmeldung: 04.06.2014
(73) Patentinhaber: GE Sensing & Inspection Technologies GmbH, 31515 Wunstorf (DE)
(72) Erfinder: Neuser, Eberhard, 31515 Wunstorf (DE); Suppes, Alexander, 30823 Garbsen (DE); Rothe, Nils, 30625 Hannover (DE); Pokutnev, Pavel, 30457 Hannover (DE)
(74) Vertreter: Patentanwälte Bauer Vorberg Kayser

(56) Entgegenhaltungen:
- EP-A1- 0 021 366
- EP-A2- 0 479 618
- WO-A1-00/00086
- WO-A1-2012/062543
- DE-A1- 19 509 007
- DE-A1-102005 033 187
- DE-A1-102011 075 527
- US-A- 4 872 187
- US-A- 5 442 674
- US-A1- 2003 118 227
- US-A1- 2005 094 771

## Beschreibung

Die Erfindung betrifft ein Verfahren zum Erfassen geometrischer Abbildungseigenschaften eines Flachbilddetektors in einer Röntgenprüfanlage zur zerstörungsfreien Werkstoffprüfung.

Die Prüfgenauigkeit einer Röntgenprüfanlage zur zerstörungsfreien Werkstoffprüfung mit einem Flachbilddetektor hängt unter anderem davon ab, dass das in der Rekonstruktion und Auswertung zugrunde gelegte geometrische Modell des Detektors möglichst gut mit den Abmessungen des realen Detektors übereinstimmt. Entgegen früherer Annahmen hat sich herausgestellt, dass Flachbilddetektoren (flat panel detectors) im Rahmen der angestrebten hohen Prüfgenauigkeiten nicht flach sind, sondern eine Krümmung bzw. Wölbung der Detektoroberfläche aufweisen. Denkbar ist beispielsweise auch, dass die Pixelgröße von Flachbilddetektoren nicht konstant, sondern eine Funktion des Ortes ist, d.h. von der Zeilen- und Spaltennummer abhängt. Dies führt zu Verzeichnungsfehlern, d.h. die Koordinate der realen Abbildung eines Merkmals, z.B. eines Punktes, im Vergleich zu der idealen Abbildung ist aufgrund der Wölbung oder nicht konstanten Pixelgröße verschoben.

Zur Korrektur von Verzeichnungsfehlern eines Flachbilddetektors schlägt die WO 2012 062543 A2 ein Verfahren zum Betreiben einer Messanordnung für einen Computertomographen vor, wobei ein Kalibrierkörper zwischen der Strahlungsquelle und dem Flachbilddetektor angeordnet und mit dem Flachbilddetektor mindestens ein Röntgenbild des Kalibrierobjekts aufgenommen und aus bekannten Abmessungen des Kalibrierkörpers und aus dem mindestens einen Röntgenbild ein Verzeichnungsfehler des Flachbilddetektors als Funktion des Ortes ermittelt wird. Der Kalibrierkörper weist eine Vielzahl von separaten Strukturen, beispielsweise Kugeln, auf, deren Abmessungen, d.h. Größe und Abstände, genau bekannt sein müssen. Dies erfordert in der Regel eine hochgenaue Vermessung des Kalibrierkörpers beispielsweise mittels eines Koordinatenmessgeräts, was entsprechend zeit- und kostenaufwändig ist.

In der DE 10 2011 075 527 A1 wird ein Verfahren zur Korrektur von verzeichnungsfehlern, die aufgrund von Lagefehlern des Flachbilddetektors indem Achsensystem des Durchstrahlungssystems relativ zu einer Solllage resultieren, offenbart. Dazu wird eine geometrische Korrektur des Durchstrahlungsrohbildes durchgeführt, so dass eine Veränderung einer Projektion des durchstrahlten Objekts in dem Durchstrahlungsrohbild aufgrund des Lagefehlers verringert beziehungsweise korrigiert wird.

Die Aufgabe der Erfindung besteht darin, ein Verfahren, eine Röntgenprüfanlage und einen Kalibrierkörper bereitzustellen, die mit einfachen Mitteln eine genaue Erfassung geometrischer Abbildungseigenschaften des Flachbilddetektors gestatten, um eine Korrektur eines entsprechenden Verzeichnungsfehlers zu ermöglichen und somit die Messgenauigkeit der Röntgenprüfanlage zu verbessern.

Die Erfindung löst diese Aufgabe mit den Merkmalen der unabhängigen Ansprüche. Die Erfindung hat erkannt, dass für die Ermittlung des ortsabhängigen Verzeichnungsfehlers die Kenntnis von Abmessungen des Kalibrierkörpers nicht erforderlich ist. Vielmehr sind hierfür Merkmale der mindestens einen diskreten geometrischen Figur ausreichend, die von den Abmessungen des Kalibrierkörpers unabhängig sind, ohne dass daraus eine Beeinträchtigung der Kalibriergenauigkeit resultiert. Dies hat gegenüber herkömmlichen Verfahren den enormen Vorteil, dass eine vorherige aufwändige, hochgenaue Vermessung des Kalibrierkörpers beispielsweise mittels eines Koordinatenmessgeräts entfallen kann.

Für die Ermittlung des ortsabhängigen Verzeichnungsfehlers sind unterschiedlichste Merkmale der mindestens einen diskreten geometrischen Figur geeignet. Verschiedene bevorzugte Merkmale werden im Folgenden erläutert.

In einer bevorzugten Ausführungsform wird durch Abbildung des mindestens einen diskreten geometrischen Objekts des Kalibrierkörpers eine Mehrzahl von diskreten geometrischen Figuren an verschiedenen Positionen aufgenommen. Dies kann insbesondrere mittels eines Kalibrierkörpers geschehen, der eine Mehrzahl von diskreten geometrischen Objekten aufweist, die gleichzeitig mit der Röntgenvorrichtung abgebildet werden können. Alternativ ist es beispielsweise auch möglich, dass der Kalibrierkörper nur ein diskretes geometrisches Objekt aufweist, das nacheinander auf verschiedene Positionen der Detektoroberfläche mit der Röntgenvorrichtung abgebildet wird.

Vorteilhaft können die den geometrischen Figuren zugrundeliegenden geometrischen Objekte gleichartig sein, wobei ein der. Ermittlung des ortsabhängigen Verzeichnungsfehlers zugrundeliegendes Merkmal die Abweichung der geometrischen Figuren von der Gleichartigkeit ist. Beispielsweise können die den geometrischen Figuren zugrundeliegenden geometrischen Objekte gleich groß sein, wobei ein der Ermittlung des ortsabhängigen Verzeichnungsfehlers zugrundeliegendes Merkmal eine untereinander abweichende Abmessung der geometrischen Figuren ist. In dieser Variante ist es lediglich erforderlich, dass die den geometrischen Figuren zugrundeliegenden geometrischen Objekte des Prüfkörpers mit hoher Genauigkeit gleich groß sind, die Kenntnis der Größe selbst ist jedoch nicht erforderlich. Die den geometrischen Figuren zugrundeliegenden geometrischen Objekte können beispielsweise auch die gleiche Form haben, wobei ein der Ermittlung des ortsabhängigen Verzeichnungsfehlers zugrundeliegendes Merkmal die untereinander abweichende Form der geometrischen Figuren ist. In einer weiteren Ausführungsform können die den geometrischen Figuren zugrundeliegenden geometrischen Objekte regelmäßig und/oder periodisch angeordnet sein, wobei ein der Ermittlung des ortsabhängigen Verzeichnungsfehlers zugrundeliegendes Merkmal die von der Regelmäßigkeit und/oder der Periodizität abweichende Anordnung der geometrischen Figuren ist.

Es ist nicht zwingend erforderlich, dass eine Mehrzahl von diskreten geometrischen Figuren an verschiedenen Positionen der Detektoroberfläche aufgenommen wird. Denkbar sind auch Ausführungsformen, bei denen nur eine einen erheblichen Bereich der Detektoroberfläche erfassende diskrete geometrische Figur aufgenommen wird.

In einer Ausführungsform kann das der geometrischen Figur zugrundeliegende geometrische Objekt mindestens eine gerade Linie oder in mindestens einer geraden Linien angeordnet sein, wobei ein der Ermittlung des ortsabhängigen Verzeichnungsfehlers zugrundeliegendes Merkmal die Abweichung der geometrischen Figur von der Geradlinigkeit ist. In einer anderen Ausführungsform kann das der geometrischen Figur zugrundeliegende geometrische Objekt mindestens ein zylindrisches Objekt mit konstantem Durchmesser sein, wobei ein der Ermittlung des ortsabhängigen Verzeichnungsfehlers zugrundeliegendes Merkmal eine Abweichung der geometrischen Figur von dem konstanten Durchmesser ist.

Der ortsabhängige Verzeichnungsfehler wird nicht aus einem oder mehreren zweidimensionalen Durchstrahlungsbildern, sondern aus einem dreidimensionalen, insbesondere mittels Computertomographie rekonstruierten Röntgenbild ermittelt. Dies hat insbesondere in einer Computertomographie-Anlage den Vorteil, dass die vorhandenen Rekonstruktionsalgorithmen auch für die Kalibrierung verwendet werden können. Des Weiteren ermöglicht ein dreidimensionales, insbesondere mittels Computertomographie rekonstruiertes Röntgenbild eine genauere Ermittlung des ortsabhängigen Verzeichnungsfehlers.

Die Erfindung wird im Folgenden anhand bevorzugter Ausführungsformen unter Bezugnahme auf die beigefügten Figuren erläutert. Dabei zeigt:
- Fig. 1: eine schematische Darstellung eines Computertomografie-Systems;
- Fig. 2: einen schematischen Längsschnitt durch einen Kalibrierkörper in einer Ausführungsform;
- Fig. 3: eine schematische Wiedergabe eines Röntgenbildes eines Kalibrierkörpers entsprechend Figur 2;
- Fig. 4: eine Darstellung des Durchmesserverlaufs der Kugeln des Kalibrierkörpers über der Detektorerstreckung;
- Fig. 5: eine zweidimensionale Darstellung des Durchmesserverlaufs der Kugeln des Kalibrierkörpers über beiden Detektorerstreckungen;
- Fig. 6: einen schematischen Längsschnitt durch einen Kalibrierkörper in einer weiteren Ausführungsform;
- Fig. 7: einen schematischen Querschnitt durch den Kalibrierkörper aus Figur 6;
- Fig. 8: eine schematische Wiedergabe eines Röntgenbildes eines Kalibrierkörpers entsprechend Figur 6;
- Fig. 9: eine schematische Darstellung eines Kalibrierkörpers in einer alternativen Ausführungsform; und
- Fig. 10: eine schematische Wiedergabe eines Röntgenbildes eines Kalibrierkörpers entsprechend Figur 9.

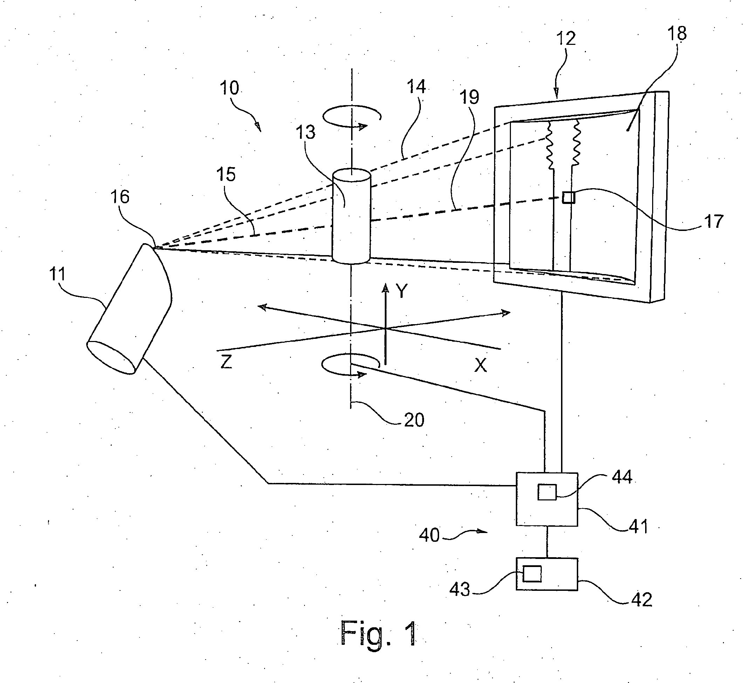

Das in Figur 1 gezeigte Computertomografie (CT)-System umfasst eine Röntgenvorrichtung 10 zum Aufnehmen von Röntgenprojektionen eines Probenkörpers 13. Zu diesem Zweck umfasst die Röntgenvorrichtung 10 eine Röntgenquelle 11, insbesondere eine Röntgenröhre, die einen Röntgenstrahlungskegel 14 emittiert, und einen bildgebenden Röntgendetektor 12. Des Weiteren ist ein nur schematisch angedeuteter Probenmänipulator 20 vorgesehen, der vorzugsweise dazu eingerichtet ist, den Probenkörper 13 um eine vertikale Achse zu drehen. Alternativ kann die Röntgenvorrichtung 10 um den feststehenden Probenkörper 13 rotiert werden. Vorzugsweise kann der Probenkörper 13 mittels des Probenmanipulators 20 in x-, y- und/oder z-Richtung linear verschoben werden. Im Allgemeinen sind die Röntgenvorrichtung 10 und der Probenkörper 13 relativ zueinander in geeigneter Weise verstellbar, was Rotation und/oder Translation jeweils um eine oder mehrere Achsen umfasst.

Der bildgebende Röntgendetektor 12 ist ein Flachbild (flat panel)-Detektor, d.h. ein Festkörper- bzw. Halbleiterdetektor, der in einer Ausführungsform eine Szintillationsschicht zum Umwandeln der einfallenden Röntgenstrahlung in Licht und eine lichtempfindliche Schicht insbesondere aus Fotozellen bzw. Fotodioden zum Umwandeln des Lichts in ein elektrisches Signal umfasst. In einer anderen Ausführungsform ist anstelle einer Szintillationsschicht und einer lichtempfindlichen Schicht ein auf Röntgenstrahlung empfindlicher Fotoleiter, beispielsweise auf der Grundlage von Selen, vorgesehen.

Ein Satz von Röntgenprojektionen des Probenkörpers 13 wird aufgenommen, indem der Manipulator 20 schrittweise um einen kleinen Winkelschritt gedreht wird und bei jeder Winkelstellung eine Röntgenprojektion aufgenommen wird. Eine Röntgenprojektion 18 wie beispielsweise in Figur 1 gezeigt ist eine zweidimensionale Abbildung, wobei der detektierte Dichtewert eines Pixels 17, typischerweise ein Grauwert, die Schwächung des entsprechenden Röntgenstrahls 15 von dem Fokalpunkt 16 der Röntgenquelle 11 durch den Probenkörper 13 wiedergibt, wodurch ein geschwächter Röntgenstrahl 19 zu dem entsprechenden Pixel 17 resultiert. Aufgrund einer Wölbung der empfindlichen Oberfläche des Detektors 12 ist es möglich, dass ein Objektpunkt nicht auf das ideale Pixel 17, sondern auf eine andere Position bzw. ein anderes Pixel abgebildet wird.

Die aufgenommenen Röntgenprojektionen werden aus dem Röntgendetektor 12 ausgelesen und zu einer Computervorrichtung 40 übermittelt, wo sie in einem Speicher 44 zur weiteren Auswertung und Verarbeitung gespeichert werden. Die Computervorrichtung 40 umfasst einen programmierbaren Computer 41 insbesondere mit einem Mikroprozessor oder Microcontroller, und ein Bedienterminal 42 mit einem Bildschirm 43. Der Computer 41 umfasst eine Software zur Durchführung eines geeigneten CT-Rekonstruktionsalgorithmus, um aus den aufgenommenen Röntgenprojektionen ein dreidimensionales rekonstruiertes Bild (Volumenbild) des Probenkörpers 13 zu ermitteln. Alternativ kann zur Durchführung der Rekonstruktion ein separater Computer vorgesehen sein. In dem Ausführungsbeispiel gemäß Figur 1 ist der Computer 41 zur Steuerung der Röntgenvorrichtung 10, insbesondere der Röntgenquelle 11, des Röntgendetektors 12 und des Probenmanipulators 20 eingerichtet. Alternativ kann eine separate Steuereinrichtung zur Steuerung der Röntgenvorrichtung 10 vorgesehen sein.

Zur Kalibrierung des Flachbilddetektors 12 wird ein Kalibrierkörper 13 in den Strahlengang 14 der Röntgenvorrichtung 10 eingebracht, sodann werden Röntgenbilder bzw. -projektionen des Kalibrierkörpers 13 aufgenommen und die Volumendichte des Kalibrierkörpers 13 rekonstruiert.

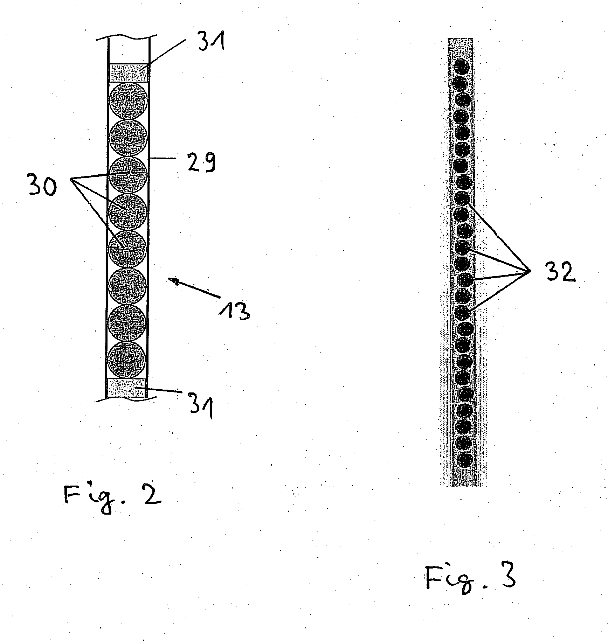

Eine Ausführungsform eines Kalibrierkörpers 13 ist in Figur 2 gezeigt. In einem Rohr 29 aus einem geeigneten, vorzugsweise strahlentransparenten Material, beispielsweise einem Kunststoff oder Aluminium, ist hier eine einzelne Reihe von Kugeln 30 aus einem geeigneten röntgenabsorbierenden Material, beispielsweise Stahl oder Keramik, vorzugsweise auf Kontakt zueinander angeordnet. Die Kugeln 30 bilden eine Mehrzahl von separaten, nicht miteinander verbundenen Kalibrierobjekten.

Der Innendurchmesser des Rohrs 29 ist hier größer als der Durchmesser einer Kugel 30, jedoch kleiner als der doppelte Durchmesser einer Kugel 30. An beiden Enden kann das Rohr 29 mittels vorzugsweise elastischen Verschlusskörpern 31 beispielsweise aus Schaumstoff verschlossen sein, die die Kugeln 30 in dem Rohr 29 einspannen und somit fixieren, um ein Verwackeln der Kugeln 30 zu verhindern. Die Kugeln 30 weisen mit einer gewünschten hohen Genauigkeit, die beispielsweise im Bereich von ± 1 µm liegt, dieselben Abmessungen, d.h. denselben Durchmesser auf, der jedoch zur Durchführung des Kalibrierverfahrens nicht mit derselben Genauigkeit bekannt sein muss und in der Regel nur mit einer erheblich größeren Toleranz bekannt ist. Beispielsweise kann der Durchmesser der Kugeln 30 in einem herstellungsbedingten Toleranzbereich von ± 100 µm (oder mehr) um einen Nominalwert liegen, sofern nur der Durchmesserunterschied der Kugeln 30 untereinander klein ist (beispielsweise im Bereich von ± 1 µm). Beispielsweise ist es möglich, vergleichsweise kostengünstige Kugellager-Kugeln als Kalibrierobjekte 30 zu verwenden. Zwar kann der Durchmesser von Kugellager-Kugeln von einer Charge zur nächsten erheblich schwanken, jedoch haben Kugellager-Kugeln innerhalb einer Charge gewöhnlich mit sehr hoher Genauigkeit denselben Durchmesser, der jedoch nicht genau bekannt ist. Aus diesem Grund können für das vorliegende Kalibrierverfahren als Kalibrierobjekte 30 ohne Weiteres beispielsweise Kugellager-Kugeln aus derselben Charge verwendet werden; eine hochgenaue Vermessung des Durchmessers der Kugeln 30 ist dabei nicht erforderlich.

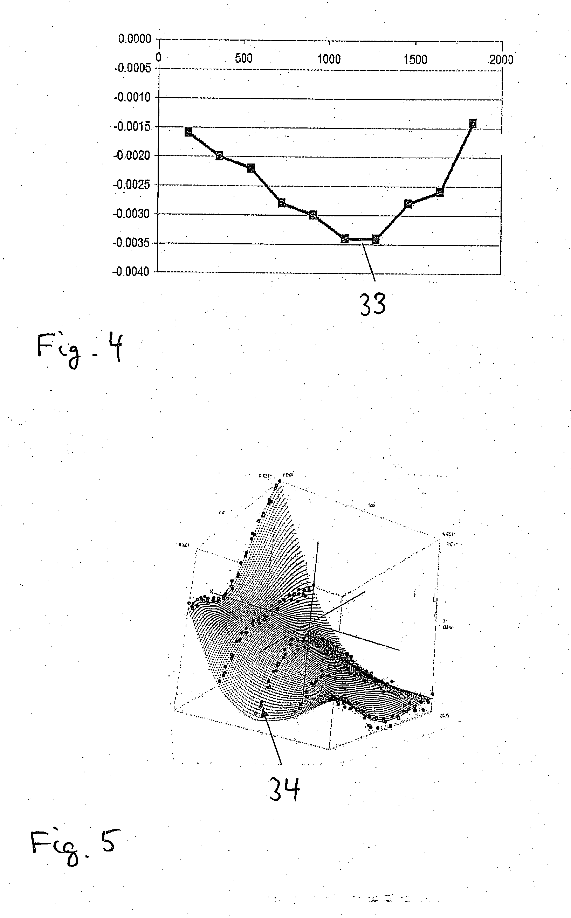

Der Kalibrierkörper 13 wird vorteilhaft entlang oder parallel zu der Drehachse (vertikale Achse bzw. y-Achse in Figur 1) angeordnet, so dass die Kugeln 30 über die Erstreckung (hier die Höhe) des Detektors 12 verteilt sind, sodann werden Röntgenprojektionen aufgenommen. Ein Röntgenbild bzw. eine Röntgenprojektion des Kalibrierkörpers 13 aus Figur 2 ist beispielsweise in Figur 3 gezeigt. Die Reihe von Kugeln 30 des Kalibrierkörpers 13 führt zu einer entsprechenden Reihe von Kugelfiguren 32 im Röntgenbild. In dem Computer 41 wird aus sämtlichen Projektionen ein Volumenbild des Kalibrierkörpers 13 mit dreidimensionalen Kugelfiguren 32 rekonstruiert. Aus dem rekonstruierten Volumenbild wird der Durchmesser der dreidimensionalen Kugelfiguren 32 mittels Auswertung bzw. Bildverarbeitung im Computer 41 bestimmt. Der relative Durchmesserverlauf 33 der dreidimensionalen Kugelfiguren 32 ist beispielsweise in Figur 4 über der Kugelreihe (hier zehn Datenpunkte entsprechend zehn Kugeln 30) aufgetragen. Genauer gesagt zeigt Figur 4 die relative Durchmesserabweichung 33 beispielsweise in mm über der Detektorerstreckung (hier Höhe) beispielsweise in Pixeln. Da die Durchmesserabweichung bzw. der Durchmesserverlauf nicht konstant ist, lässt dies nach einer Zuordnung zu den entsprechenden Detektorpositionen Rückschlüsse auf die Detektorwölbung zu.

Vergleichbare Messungen werden vorteilhaft über die gesamte oder einen erheblichen Teil der sensitiven Oberfläche des Detektors 12 durchgeführt, indem der Kalibrierkörper 13 sukzessive senkrecht zu seiner Längserstreckung verschoben und entsprechende Röntgenbilder aufgenommen werden. Wenn der Kälibrierkörper 13 beispielsweise parallel zu der Drehachse angeordnet ist, erfolgt die Verschiebung zweckmäßigerweise senkrecht zur Drehachse. Die resultierende zweidimensionale Durch-messerabweichung 34 beispielsweise in mm über der Detektorhöhe und -breite beispielsweise jeweils in Pixeln ist in Figur 5 gezeigt. Daraus kann die zweidimensionale Wölbung des Detektors 12 ermittelt werden. Aus der Wölbung des Detektors 12 kann ein ortsabhängiger Verzeichnungsfehler, d.h. ein pixelgenauer Verzeichnungsfehler in Abhängigkeit von der x- und y-Koordinate jedes Pixels, ermittelt und insbesondere in dem Computer 41 gespeichert werden. Jede nachfolgend gemessene Röntgenprojektion kann dann auf den ermittelten Verzeichnungsfehler korrigiert werden, wodurch die Genauigkeit der gemessenen Projektionen und somit auch der rekonstruierten Daten signifikant gesteigert werden kann. Alternativ kann der Verzeichnungsfehler während der Rekonstruktion berücksichtigt werden, ohne dass die Röntgenprojektionen selbst korrigiert werden müssen. Zusätzlich oder alternativ zur Wölbung der sensitiven Detektoroberfläche kann die Pixelgröße bzw. die lokale Pixelgrößenabweichung pixelgenau bestimmt werden.

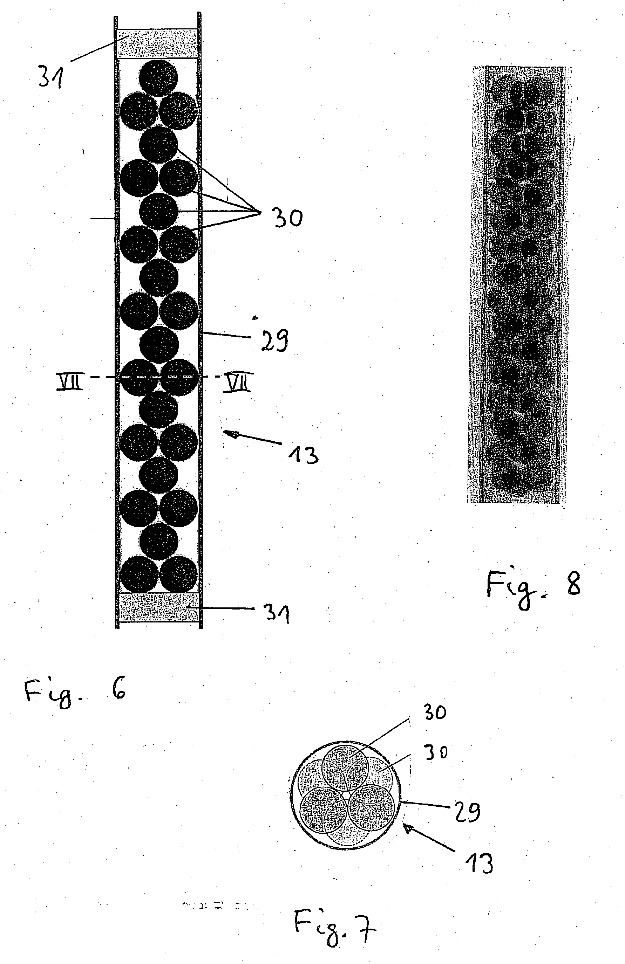

Eine alternative Ausführung des Kalibrierkörpers 13 ist in den Figuren 6 und 7 gezeigt. Der Innendurchmesser des Rohrs 29 ist hier größer als der zweifache, vorzugsweise größer als der 2,1547-fache Durchmesser einer Kugel 30. Auf diese Weise können in einer zur Drehachse senkrechten Ebene eine Mehrzahl von Kugeln 30, vorzugsweise mindestens drei Kugeln 30, angeordnet werden. In der nächsten Kugelebene ist die Dreier-Kugelgruppe um 60° verdreht, wie aus dem Querschnitt gemäß Figur 7 ersichtlich ist. Der Vorteil dieser Ausführungsform liegt darin, dass für jede axiale Position mehrere Messwerte zur Verfügung stehen, die eine Mittelung und somit eine Steigerung der Messgenauigkeit erlauben. Es sind auch Ausführungsformen mit zwei Kugeln, oder mehr als drei Kugeln, pro zur Drehachse senkrechter Ebene möglich.

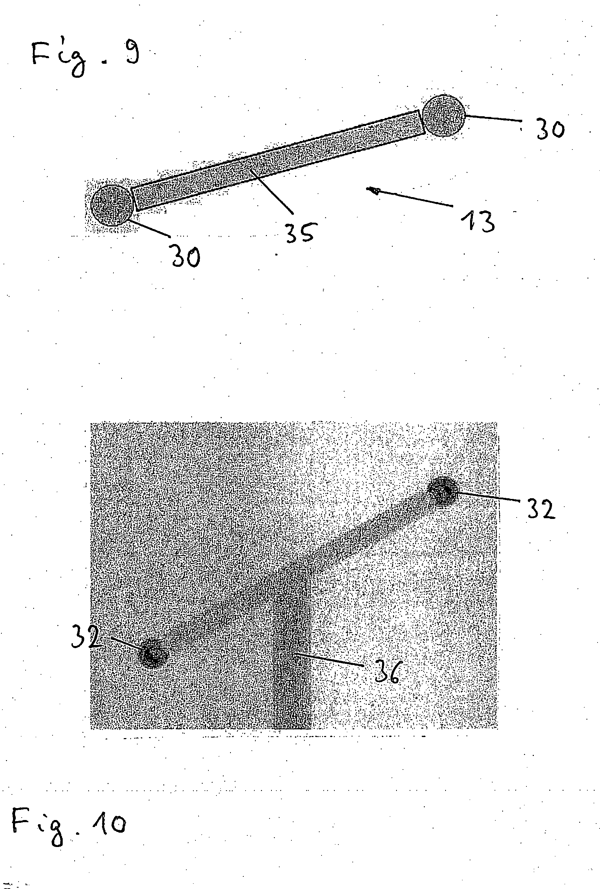

Eine weitere Ausführung des Kalibrierkörpers 13 ist in den Figuren 9 und 10 gezeigt. Der Kalibrierstab 13 umfasst hier einen vorzugsweise röntgenstransparenten Stab 35 beispielsweise aus CFK, an dessen Enden jeweils ein hier kugelförmiges, röntgenabsorbierendes Kalibrierobjekt 30 aus einem geeigneten Material, beispielsweise Rubin, angebracht ist. Die Länge des Stabes muss nicht bekannt sein und liegt beispielsweise im Bereich zwischen 2 mm und 200 mm. Der Kalibrierstab 13 kann auf unterschiedlichen Positionen des Detektors 12 aufgenommen werden und aus den relativen Abeichungen der Längen zueinander kann auf eine Wölbung des Detektors 12 geschlossen werden. In einem in Figur 10 gezeigten entsprechenden Röntgenbild ist noch ein in Figur 9 nicht gezeigter Halter 36 zu sehen.

Die Anmeldung ist vorzugsweise auf Prüfanlagen für die zerstörungsfreie Prüfung von nichtbiologischen Prüfkörpern anwendbar.

Der Kalibrierkörper 13 ist nicht auf die gezeigten Ausführungsformen beschränkt. Beispielsweise kann es sich alternativ um einen plattenförmigen Kalibrierkörper 13 in Form einer Maske mit Kalibrierobjekten beispielsweise in Form von parallelen oder gitterförmig angeordneten Linien handeln, wobei im Röntgenbild Abweichungen der Linien von der Geradlinigkeit ermittelt werden. Die Kalibrierobjekte können beispielsweise auch Kreise, Kreuze oder dergleichen sein, die den gleichen Abstand voneinander haben, der jedoch nicht bekannt sein muss. Eine andere Ausführungsform ist ein Zylinder beispielsweise aus Stahl mit hoher Durchmesserkonstanz entlang der Achse, wobei im Röntgenbild Abweichungen des Durchmessers entlang der Zylinderachse ermittelt werden. Vielfältige alternative Ausführungsformen des Kalibrierkörpers 13 sind denkbar.

## Patentansprüche

1. Verfahren zum Erfassen geometrischer Abbildungseigenschaften eines Flachbilddetektors (12) in einer Röntgenprüfanlage, umfassend die Schritte
- Anordnen eines Kalibrierkörpers (13) zwischen einer Röntgenstrahlungsquelle (11) und dem Flachbilddetektor (12), wobei der Kalibrierkörper (13) mindestens ein diskretes geometrisches Objekt (30) aufweist;
- Aufnehmen mehrerer Röntgenbilder des Kalibrierkörpers (13) mit dem Flachbilddetektor (12), wobei in den Röntgenbildern durch Abbildung des mindestens einen diskreten geometrischen Objekts (30) des Kalibrierkörpers (13) mindestens eine diskrete geometrische Figur (32) erzeugt wird;
**dadurch gekennzeichnet, dass**
die Ermittlung eines ortsabhängigen Verzeichnungsfehlers des Flachbilddetektors (12) aus einem dreidimensionalen Röntgenbild erfolgt, das aus den mehreren Röntgenbildern (32) rekonstruiert wird, wobei die Ermittlung des ortsabhängigen Verzeichnungsfehlers auf der Grundlage mindestens eines Merkmals der mindestens einen diskreten geometrischen Figur erfolgt, undsämtliche zur Ermittlung des ortsabhängigen Verzeichnungsfehlers verwendeten Merkmale der mindestens einen diskreten geometrischen Figur (32) von den Abmessungen des Kalibrierkörpers (13) unabhängig sind.

2. Verfahren nach Anspruch 1, wobei durch Abbildung des mindestens einen diskreten geometrischen Objekts (30) des Kalibrierkörpers (13) eine Mehrzahl von diskreten geometrischen Figuren (32) an verschiedenen Positionen der Detektoroberfläche aufgenommen werden.

3. Verfahren nach Anspruch 2, **dadurch gekennzeichnet, dass** die den geometrischen Figuren (32) zugrundeliegenden geometrischen Objekte (30) gleichartig sind und ein Merkmal die Abweichung der geometrischen Figuren (32) von der Gleichartigkeit ist.

4. Verfahren nach Anspruch 2 oder 3, **dadurch gekennzeichnet, dass** die den geometrischen Figuren (32) zugrundeliegenden geometrischen Objekte (30) gleich groß sind und ein Merkmal eine untereinander abweichende Abmessung der geometrischen Figuren (32) ist.

5. Verfahren nach einem der Ansprüche 2 bis 4, **dadurch gekennzeichnet, dass** die den geometrischen Figuren (32) zugrundeliegenden geometrischen Objekte (30) die gleiche Form haben und ein Merkmal die untereinander abweichende Form der geometrischen Figuren (32) ist.

6. Verfahren nach einem der Ansprüche 2 bis 5, **dadurch gekennzeichnet, dass** die den geometrischen Figuren (32) zugrundeliegenden geometrischen Objekte (30) regelmäßig und/oder periodisch angeordnet sind und ein Merkmal die von der Regelmäßigkeit und/oder der Periodizität abweichende Anordnung der geometrischen Figuren (32) ist.

7. Verfahren nach einem der Ansprüche 2 bis 6, **dadurch gekennzeichnet, dass** die den geometrischen Figuren (32) zugrundeliegenden geometrischen Objekte (30) auf Kontakt zueinander angeordnet sind.

8. Verfahren nach einem der vorangehenden Ansprüche, **dadurch gekennzeichnet, dass** das der geometrischen Figur (32) zugrundeliegende geometrische Objekt mindestens eine gerade Linie ist oder in mindestens einer geraden Linien angeordnet ist und ein Merkmal die Abweichung der geometrischen Figur (32) von der Geradlinigkeit ist.

9. Verfahren nach einem der vorangehenden Ansprüche, **dadurch gekennzeichnet, dass** das der geometrischen Figur (32) zugrundeliegende geometrische Objekt mindestens ein zylindrisches Objekt mit konstantem Durchmesser ist und ein Merkmal eine Abweichung der geometrischen Figur (32) von dem konstanten Durchmesser ist.

10. Röntgenprüfanlage mit einer Röntgenstrahlungsquelle (11), einem Flachbilddetektor (12), einem Kalibrierkörper (13), der mindestens ein diskretes geometrisches Objekt (30) aufweist, und einer elektronischen Datenverarbeitungseinrichtung (41), wobei die Röntgenprüfanlage zum Aufnehmen mehrerer Röntgenbilder des zwischen der Röntgenstrahlungsquelle (11) und dem Flachbilddetektor (12) angeordneten Kalibrierkörpers (13) eingerichtet ist, wobei in den Röntgenbildern durch Abbildung des mindestens einen diskreten geometrischen Objekts (30) des Kalibrierkörpers (13) mindestens eine diskrete geometrische Figur (32) erzeugt wird, wobei die Datenverarbeitungseinrichtung (41) zur Rekonstruktion eines dreidimensionalen Röntgenbildes aus den mehreren Röntgenbildern eingerichtet ist, **dadurch gekennzeichnet, dass** die Datenverarbeitungseinrichtung zur Ermittlung eines ortsabhängigen Verzeichnungsfehlers des Flachbilddetektors (12) aus dem dreidimensionalen Röntgenbild auf der Grundlage mindestens eines Merkmals der diskreten geometrischen Figur (32) eingerichtet ist, wobei sämtliche zur Ermittlung des ortsabhängigen Verzeichnungsfehlers verwendeten Merkmale der mindestens einen diskreten geometrischen Figur (32) von den Abmessungen des Kalibrierkörpers (13) bzw. deren genauer Kenntnis unabhängig sind.

11. Röntgenprüfanlage gemäß Anspruch 10, **dadurch gekennzeichnet, dass** der Kalibrierkörper (13) ein zylindrisches Rohr (29) und eine Reihe von in dem zylindrischen Rohr (29) angeordnete, gleichartige geometrische Objekte (30) umfasst.

12. Verwendung eines Kalibrierkörpers (13) zum Erfassen geometrischer Abbildungseigenschaften eines Flachbilddetektors (12) in einem Verfahren gemäß Anspruch 1, oder als Kalibrierkörper einer Röntgenprüfanlage gemäß Anspruch 10, wobei der Kalibrierkörper ein zylindrisches Rohr (29) und eine Reihe von in dem zylindrischen Rohr (29) angeordnete, gleichartige geometrische Objekte (30) umfasst.

13. Verwendung eines Kalibrierkörpers nach Anspruch 12, **dadurch gekennzeichnet, dass** die geometrischen Objekte (30) Kugeln gleichen Durchmessers sind.

14. Verwendung eines Kalibrierkörpers nach Anspruch 12 oder 13, **dadurch gekennzeichnet, dass** die geometrischen Objekte (30) nicht miteinander verbunden und/oder auf Kontakt zueinander angeordnet sind.

15. Verwendung eines Kalibrierkörpers nach einem der Ansprüche 12 bis 14, **dadurch gekennzeichnet, dass** in einer Ebene senkrecht zur Rohrachse eine Mehrzahl von geometrischen Objekten (30), vorzugsweise mindestens drei geometrische Objekte, angeordnet sind.

## Claims

1. A method for detecting geometrical imaging properties of a flat panel detector (12) in an X-ray testing system, comprising the steps of:
- disposing a calibrating body (13) between an X-ray beam source (11) and the flat panel detector (12), the calibrating body (13) comprising at least one discrete geometrical object (30);
- recording several X-ray images of the calibrating body (13) with the flat panel detector (12), with at least one discrete geometrical figure (32) being generated in the X-ray images by imaging the at least one discrete geometrical object (30) of the calibrating body (13); **characterized in that** a positionally dependent distortion error of the flat panel detector (12) is determined from a three-dimensional X-ray image reconstructed from the several X-ray images (32), wherein the determination of the positionally dependent distortion error takes place based on at least one feature of the at least one discrete geometrical figure, and all features of the at least one discrete geometrical figure (32) used for determining the positionally dependent distortion error are independent from the dimensions of the calibrating body (13).

2. The method according to claim 1, wherein a plurality of discrete geometrical figures (32) is recorded at different positions of the detector surface by imaging the at least one discrete geometrical object (30) of the calibrating body (13).

3. The method according to claim 2, **characterized in that** the geometrical objects (30) underlying the geometrical figures (32) are similar, and the deviation of the geometrical figures (32) from the similarity is a feature.

4. The method according to claim 2 or 3, **characterized in that** the geometrical objects (30) underlying the geometrical figures (32) are of the same size, and dimensions of the geometrical figures (32) deviating from one another is a feature.

5. The method according to any one of the claims 2 to 4, **characterized in that** the geometrical objects (30) underlying the geometrical figures (32) have the same shape, and the shape of the geometrical figures (32) deviating from one another is a feature.

6. The method according to any one of the claims 2 to 5, **characterized in that** the geometrical objects (30) underlying the geometrical figures (32) are arranged regularly and/or periodically, and the arrangement of the geometrical figures (32) deviating from the regularity and/or periodicity is a feature.

7. The method according to any one of the claims 2 to 6, **characterized in that** the geometrical objects (30) underlying the geometrical figures (32) are disposed so as to contact each other.

8. The method according to any one of the preceding claims, **characterized in that** the geometrical object underlying the geometrical figure (32) is at least one straight line or is disposed in at least one straight line, and the deviation of the geometrical figure (32) from the rectilinearity is a feature.

9. The method according to any one of the preceding claims, **characterized in that** the geometrical object underlying the geometrical figure (32) is at least one cylindrical object with a constant diameter, and the deviation of the geometrical figure (32) from the constant diameter is a feature.

10. An X-ray testing system with an X-ray beam source (11), a flat panel detector (12), a calibrating body (13) comprising at least one discrete geometrical object (30), and an electronic data processing device (41), wherein the X-ray testing system is configured for recording several X-ray images of the calibrating body (13) disposed between the X-ray beam source (11) and the flat panel detector (12), wherein at least one discrete geometrical figure (32) is generated in the X-ray images by imaging the at least one discrete geometrical object (30) of the calibrating body (13), wherein the data processing device (41) is configured for reconstructing a three-dimensional X-ray image from the several X-ray images, **characterized in that** the data processing device is configured for determining a positionally dependent distortion error of the flat panel detector (12) from the three-dimensional X-ray image based on at least one feature of the discrete geometrical figure (32), wherein all features of the at least one discrete geometrical figure (32) used for determining the positionally dependent distortion error are independent from the dimensions of the calibrating body (13) or the exact knowledge thereof.

11. The X-ray testing system according to claim 10, **characterized in that** the calibrating body (13) comprises a cylindrical tube (29) and a row of similar geometrical objects (30) disposed in the cylindrical tube (29).

12. A use of a calibrating body (13) for detecting geometrical imaging properties of a flat panel detector (12) in a method according to claim 1, or as a calibrating body of an X-ray testing system according to claim 10, wherein the calibrating body comprises a cylindrical tube (29) and a row of similar geometrical objects (30) disposed in the cylindrical tube (29).

13. The use of a calibrating body according to claim 12, **characterized in that** the geometrical objects (30) are spheres of the same diameter.

14. The use of a calibrating body according to claim 12 or 13, **characterized in that** the geometrical objects (30) are not interconnected and/or disposed so as to contact each another.

15. The use of a calibrating body according to any one of the claims 12 to 14, **characterized in that** a plurality of geometrical objects (30), preferably at least three geometrical objects, are disposed in a plane perpendicular to the tube axis.

## Revendications

1. Procédé de détection de propriétés d'imagerie géométriques d'un détecteur à panneau plat (12) dans une installation de contrôle aux rayons X, comprenant les étapes consistant à
- disposer un corps de calibrage (13) entre une source de rayons X (11) et le détecteur à panneau plat (12), ledit corps de calibrage (13) présentant au moins un objet géométrique discret (30);
- prendre une pluralité d'images de rayons X du corps de calibrage (13) au moyen du détecteur à panneau plat (12), dans les images de rayons X étant générée au moins une figure géométrique discrète (32) en imageant ledit au moins un objet géométrique discret (30) du corps de calibrage (13);
**caractérisé par le fait que**
la détermination d'une distorsion dépendant du lieu, du détecteur à panneau plat (12) se fait à partir d'une image de rayons X tridimensionnelle qui est reconstruite à partir de ladite pluralité d'images de rayons X (32), la détermination de la distorsion dépendant du lieu se faisant sur la base d'au moins une caractéristique de ladite au moins une figure géométrique discrète, et toutes les caractéristiques de ladite au moins une figure géométrique discrète (32) qui sont utilisées pour déterminer la distorsion dépendant du lieu étant indépendantes des dimensions du corps de calibrage (13).

2. Procédé selon la revendication 1, dans lequel, en imageant ledit au moins un objet géométrique discret (30) du corps de calibrage (13), une pluralité de figures géométriques discrètes (32) sont prises sur des positions différentes de la surface de détecteur.

3. Procédé selon la revendication 2, **caractérisé par le fait que** les objets géométriques (30) qui sont à la base des figures géométriques (32) sont similaires et qu'une caractéristique est l'écart des figures géométriques (32) par rapport à la similarité.

4. Procédé selon la revendication 2 ou 3, **caractérisé par le fait que** les objets géométriques (30) qui sont à la base des figures géométriques (32) présentent une taille identique et qu'une caractéristique est une dimension des figures géométriques (32) qui est différente entre elles.

5. Procédé selon l'une quelconque des revendications 2 à 4, **caractérisé par le fait que** les objets géométriques (30) qui sont à la base des figures géométriques (32) présentent la même forme et qu'une caractéristique est la forme des figures géométriques (32) qui est différente entre elles.

6. Procédé selon l'une quelconque des revendications 2 à 5, **caractérisé par le fait que** les objets géométriques (30) qui sont à la base des figures géométriques (32) sont disposés régulièrement et/ou périodiquement et qu'une caractéristique est la disposition des figures géométriques (32) qui diffère de la régularité et/ou de la périodicité.

7. Procédé selon l'une quelconque des revendications 2 à 6, **caractérisé par le fait que** les objets géométriques (30) qui sont à la base des figures géométriques (32) sont disposés sur contact les uns par rapport aux autres.

8. Procédé selon l'une quelconque des revendications précédentes, **caractérisé par le fait que** l'objet géométrique qui est à la base de la figure géométrique (32) est au moins une ligne droite ou est disposé en au moins une ligne droite et qu'une caractéristique est l'écart de la figure géométrique (32) par rapport à la disposition rectiligne.

9. Procédé selon l'une quelconque des revendications précédentes, **caractérisé par le fait que** l'objet géométrique qui est à la base de la figure géométrique (32) est au moins un objet cylindrique à diamètre constant et qu'une caractéristique est un écart de la figure géométrique (32) par rapport au diamètre constant.

10. Installation de contrôle aux rayons X comprenant une source de rayons X (11), un détecteur à panneau plat (12), un corps de calibrage (13) qui présente au moins un objet géométrique discret (30), et un dispositif électronique de traitement de données (41), ladite installation de contrôle aux rayons X étant adaptée pour prendre une pluralité d'images de rayons X du corps de calibrage (13) disposé entre ladite source de rayons X (11) et ledit détecteur à panneau plat (12), dans les images de rayons X étant générée au moins une figure géométrique discrète (32) en imageant ledit au moins un objet géométrique discret (30) du corps de calibrage (13), ledit dispositif de traitement de données (41) étant adapté pour reconstruire une image de rayons X tridimensionnelle à partir de ladite pluralité d'images de rayons X, **caractérisée par le fait que** ledit dispositif de traitement de données est adapté pour déterminer une distorsion dépendant du lieu, du détecteur à panneau plat (12) à partir de l'image de rayons X tridimensionnelle, sur la base d'au moins une caractéristique de ladite figure géométrique discrète (32), toutes les caractéristiques de ladite au moins une figure géométrique discrète (32) qui sont utilisées pour déterminer la distorsion dépendant du lieu étant indépendantes des dimensions du corps de calibrage (13) ou bien de la connaissance exacte de celles-ci.

11. Installation de contrôle aux rayons X selon la revendication 10, **caractérisée par le fait que** le corps de calibrage (13) comprend un tube cylindrique (29) et une série d'objets géométriques (30) similaires disposés à l'intérieur du tube cylindrique (29).

12. Utilisation d'un corps de calibrage (13) pour détecter des propriétés d'imagerie géométriques d'un détecteur à panneau plat (12) dans un procédé selon la revendication 1, ou en tant que corps de calibrage d'une installation de contrôle aux rayons X selon la revendication 10, ledit corps de calibrage comprenant un tube cylindrique (29) et une série d'objets géométriques (30) similaires disposés à l'intérieur du tube cylindrique (29).

13. Utilisation d'un corps de calibrage selon la revendication 12, **caractérisée par le fait que** les objets géométriques (30) sont des billes de diamètre identique.

14. Utilisation d'un corps de calibrage selon la revendication 12 ou 13, **caractérisée par le fait que** les objets géométriques (30) ne sont pas liés entre eux et/ou sont disposés sur contact les uns par rapport aux autres.

15. Utilisation d'un corps de calibrage selon l'une quelconque des revendications 12 à 14, **caractérisée par le fait qu'**une pluralité d'objets géométriques (30), de préférence au moins trois objets géométriques, sont disposés dans un plan perpendiculaire à l'axe du tube.
